# EUROPEAN PATENT APPLICATION

(11) **EP 4 480 393 A1**
(43) Date of publication of application: **25.12.2024**
(21) Application number: 23181031.8
(22) Date of filing: 22.06.2023
(51) Int. Cl.: A61B 5/00, A61B 5/02, A61B 18/20, A61N 5/06

(54) **SYSTEM AND METHOD FOR IMAGING AN ANATOMICAL STRUCTURE**

(71) Applicant: Koninklijke Philips N.V., 5656 AG Eindhoven (NL)
(72) Inventor: DAMODARAN, Mathivanan, 5656AG Eindhoven (NL); GERHARDT, Lutz Christian, 5656AG Eindhoven (NL); JOHNSON, Mark Thomas, 5656AG Eindhoven (NL); PAULUSSEN, Elvira Johanna Maria, 5656AG Eindhoven (NL)
(74) Representative: Philips Intellectual Property & Standards

(57) **Abstract**

A system and method for imaging an anatomical structure. Light emitted by a light source of a handheld personal care device is backscattered by the anatomical structure and detected by a light detecting arrangement of the handheld personal care device. At least one location, relative to the light source, at which light is detected by the light detecting arrangement is obtained and processed to determine a relative depth at which the detected light was backscattered by the anatomical structure.

## Description

### FIELD OF THE INVENTION

The invention relates to the field of imaging anatomical structures, and in particular, to light-based imaging of anatomical structures.

### BACKGROUND OF THE INVENTION

Deep blood vessels are found deep inside the body and are important for maintaining blood circulation to deliver oxygen and nutrients to organs and tissue throughout the body.

A wide range of problems can affect deep blood vessels, including narrowing, blockage, inflammation, or leakage. Common conditions that affect deep blood vessels include deep vein thrombosis, varicose veins and peripheral artery disease. Approximately 10-20% of the world's population suffers from such conditions, affecting around 22 million women and 11 million men in the United States of America alone.

Problems with deep blood vessels can have wider health implications. For instance, blood clots in deep arteries can increase the risk of a stroke or heart attack, and may also cause severe leg pain and difficulty in walking, in some cases resulting in loss of a limb.

Conditions affecting deep blood vessels are usually only diagnosed at a late stage. Diagnosis generally requires a physical examination or an ultrasound scan of blood flow.

There is therefore a need for improved imaging of deep blood vessels.

### SUMMARY OF THE INVENTION

The invention is defined by the claims.

According to examples in accordance with an aspect of the invention, there is provided a system for imaging an anatomical structure, the system comprising: a handheld personal care device, comprising: a light source configured to emit light; and a light detecting arrangement comprising at least one light detector at a known position relative to the light source; and a processing system configured to: obtain, from the light detecting arrangement, one or more locations on the light detecting arrangement, at which light emitted by the light source and backscattered by the anatomical structure is detected by the light detecting arrangement; and for at least one of the one or more locations at which light is detected by the light detecting arrangement, process at least the location to determine a relative depth at which the light was backscattered by the anatomical structure.

The inventors have recognized that users of handheld personal care devices use such devices on a regular basis. Thus, by analyzing the backscattering of light emitted from a handheld personal care device, changes in an anatomical structure, such as a deep blood vessel, may be identified at an early stage.

Further, handheld personal care devices, such as hair removal devices, are often used at locations in which vascular changes tend to occur.

The location at which light is detected by the light detecting arrangement may comprise which pixel of a light detector detected the light and/or which of a plurality of light detectors detected the light. Since the distance between each light detector and the light source is known, the location at which light is detected provides information about the spatial distribution of backscattered light.

The inventors have recognized that the spatial distribution of backscattered light depends on the depth at which the light was backscattered, with light backscattered at a greater depth being detected further from the light source. By determining the depth at which light is backscattered from an anatomical object, an image of (at least part of) the anatomical structure may be obtained.

The light source may be a functional part of (i.e. relating to the function of) the handheld personal care device (e.g. in the case of an IPL removal device), or may be additionally provided on the handheld personal care device for the purpose of imaging an anatomical structure.

In some examples, the processing system is further configured to obtain, for each location at which light is detected by the light detecting arrangement, a time at which light is detected at the location; and the step of determining the depth at which the light was backscattered by the anatomical structure further comprises processing at least, for each location for which a relative depth is determined, the time at which light is detected at the location.

The time at which light is detected at each location may improve a precision of the determination of depth, for example, by allowing light emitted from a part of the light source further away from the location and backscattered at a greater depth to be distinguished from light emitted from a part of the light source closer to the location and backscattered at a shallower depth, as the light backscattered at a greater depth will take longer to reach the location.

In some examples, the at least one light detector comprises at least one SPAD sensor.

The high temporal resolution of a SPAD sensor allows the time at which light is detected to be recorded with greater precision.

In some examples, the processing system is further configured to obtain, for each location at which light is detected by the light detecting arrangement, a spectrum of light detected at the location; and the step of determining the relative depth at which the light was backscattered by the anatomical structure further comprises processing, for each location for which a relative depth is determined, the spectrum of light detected at the location.

In some examples, the processing system is further configured to process, for each location for which a relative depth is determined, the relative depth at which the light was backscattered by the anatomical structure and the spectrum of light to classify the anatomical structure from which the light was backscattered.

The inventors have recognized that the spectrum of light backscattered by body tissue depends on a tissue type, and that the combination of the depth of the anatomical structure and the spectrum of backscattered light allows the identification of the anatomical structure. For instance, red light backscattered deep within the body is indicative of a deep artery.

The spectrum of light detected at the location may be obtained using a CMOS/CCD sensor as the at least one light detector in combination with a diffraction grating, or by using a wavelength filter.

In some examples, the system further comprises: a polarization conversion system configured to intercept light emitted by the light source and convert p-polarized light to s-polarized light; and a polarizing filter provided over the light detecting arrangement to filter out s-polarized light.

This arrangement reduces an amount of surface reflection detected by the light detecting arrangement.

In some examples, the processing system is further configured to: repeat, for a plurality of positions of the light source relative to the anatomical structure, the steps of: obtaining, from the light detecting arrangement, one or more locations on the light detecting arrangement, at which light emitted by the light source and backscattered by the anatomical structure is detected by the light detecting arrangement; and for at least one of the one or more locations at which light is detected by the light detecting arrangement, processing at least the location to determine a relative depth at which the light was backscattered by the anatomical structure; and process each relative depth to generate an image of the anatomical structure.

In other words, an image of the anatomical structure (e.g. a longitudinal image of a deep blood vessel) may be generated by imaging the anatomical structure from a plurality of positions.

In some examples, the processing system is further configured to: obtain, for each relative depth, a corresponding historical depth at which light was backscattered by the anatomical structure, wherein the corresponding historical depth was determined using light emitted from a same position relative to the anatomical structure; and process each relative depth and corresponding historical depth to determine, if present, a change in the anatomical structure.

For instance, where the anatomical structure is a blood vessel, a comparison with historical information allows the detection of any changes in blood vessel dilation.

In some examples, the processing system is further configured to: repeat, at a same position of the light source relative to the anatomical structure, the steps of: obtaining, from the light detecting arrangement, one or more locations on the light detecting arrangement at which light emitted by the light source and backscattered by the anatomical structure is detected by the light detecting arrangement; and for at least one of the one or more locations at which light is detected by the light detecting arrangement, processing at least the location to determine a relative depth at which the light was backscattered by the anatomical structure; and process the determined relative depths and the determined change in the anatomical structure to predict whether, if present, the change is a dynamic or permanent change.

By making several depth determinations at a single location in a single measurement session (e.g. from successive pulses of emitted light), it may be determined whether a change between a current depth and a historical depth is a dynamic change due to pulsatile blood flow or a permanent change due to disease.

In some examples, the handheld personal care device is an intense pulsed light, IPL, hair removal device; and the light source is the intense pulsed light source of the IPL hair removal device.

The high intensity of the light emitted by an IPL hair removal device means that the emitted light penetrates deep into body tissue, allowing the imaging of anatomical structures at greater depths (e.g. deep blood vessels).

In some examples, the IPL hair removal device comprises a window through which light emitted by the light source is transmitted, the window comprising two first edges of a first length and two second edges of a second, shorter length; and the light detecting arrangement is provided at a side of one or both of the first edges.

Providing the light detecting arrangement at a side of a long edge of the IPL window reduces an uncertainty in the distance between the part of the IPL window from which the light was emitted and the location at which the light was detected, thus improving a precision of the determined depth.

In some examples, the light detecting arrangement is provided outside a tissue treatment area of the handheld personal care device.

Providing the light detecting arrangement outside the treatment area enables the imaging of anatomical structures within the treatment area at greater depths.

In some examples, the anatomical structure is a blood vessel.

According to another aspect of the invention, there is provided a computer-implemented method for imaging an anatomical structure, the computer-implemented method comprising: obtaining, from a light detecting arrangement of a handheld personal care device, one or more locations, on the light detecting arrangement, at which light emitted by a light source of the handheld personal care device and backscattered by the anatomical structure is detected by the light detecting arrangement, wherein the light detecting arrangement is provided at a known position relative to the light source; and for at least one of the one or more locations at which light is detected by the light detecting arrangement, process at least the location to determine a relative depth at which the light was backscattered by the anatomical structure.

There is also proposed a computer program product comprising computer program code means which, when executed on a computing device having a processing system, cause the processing system to perform all of the steps of the computer-implemented method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for imaging an anatomical structure, according to an embodiment of the invention;
Fig. 2 illustrates the relationship between the distance between a light source and a light detector and the depth at which light detected at the light detector was backscattered by body tissue;
Fig. 3 illustrates a skin-contacting portion of an IPL hair removal device, according to an embodiment of the invention; and
Fig. 4 illustrates a computer-implemented method for imaging an anatomical structure, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for imaging an anatomical structure. Light emitted by a light source of a handheld personal care device is backscattered by the anatomical structure and detected by a light detecting arrangement of the handheld personal care device. At least one location, relative to the light source, at which light is detected by the light detecting arrangement is obtained and processed to determine a relative depth at which the detected light was backscattered by the anatomical structure.

Embodiments are at least partly based on the realization that handheld personal care devices are used against a user's skin, and that light emitted from a handheld personal care device towards the user's skin that penetrates the skin may be used to provide information anatomical structures below the surface of the skin (e.g. vascular structures), by determining the spatial distribution of backscattered light.

Illustrative embodiments may, for example, be employed in handheld personal care devices, including IPL hair removal devices, shavers, mechanical epilators, body groomers and skin treatment devices.

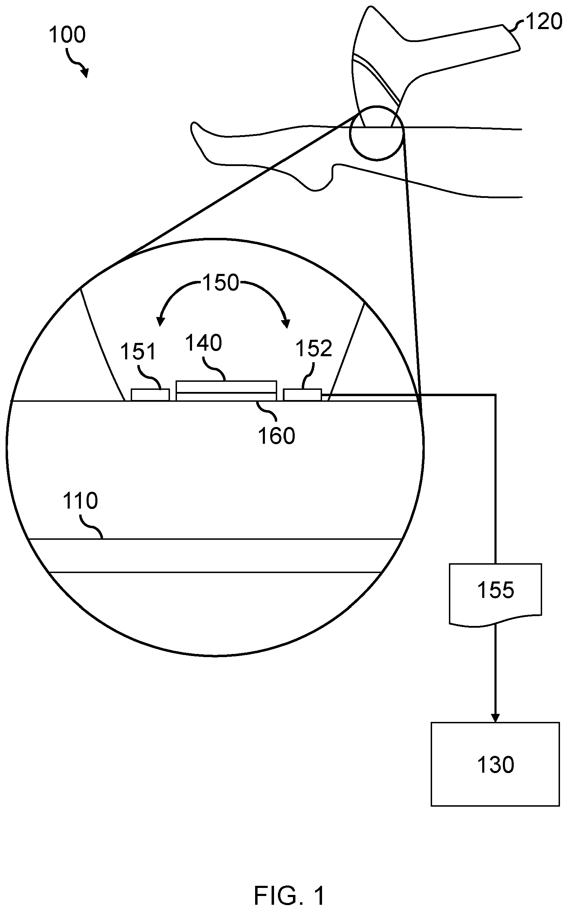

Fig. 1 illustrates a system 100 for imaging an anatomical structure 110, according to an embodiment of the invention. The system comprises a handheld personal care device 120 and a processing system 130. The handheld personal care device 120 comprises a light source 140 configured to emit light, and a light detecting arrangement 150. In Fig. 1, the processing system is shown as external to the handheld personal care device; however, in some examples, some or all of the processing system may be comprised within the handheld personal care device.

The handheld personal care device 120 of Fig. 1 is an intense pulsed light, IPL, hair removal device, that has been adapted to detect backscattered light. The light source 140 is the IPL light source already provided in an IPL hair removal device for the purpose of hair removal. In other examples, the handheld personal care device may be a device that does not use a light source to carry out its primary function, but has been adapted to include a light source and light detecting arrangement. For instance, the handheld personal care device may be a shaver, mechanical epilator or body groomer.

The light source 140 is positioned such that, during normal use of the handheld personal care device, light emitted by the light source is transmitted towards a user's skin. The light detecting arrangement 150 is positioned to detect light incident on a surface of the handheld personal care device that faces the user's skin during normal operation of the handheld personal care device.

The light detecting arrangement 150 comprises at least one light detector provided at a fixed position relative to the light source. Each light detector may comprise an array of pixels (e.g. a line array or a 2D array).

The light detecting arrangement of Fig. 1 comprises a first light detector 151 and a second light detector 152 provided on either side the light source 140. Other light detecting arrangements are also envisaged, including multiple light detectors on a same side of the light source. The light detectors 151, 152 of Fig. 1 are positioned outside a window 160 of the IPL hair removal device through which light emitted by the light source is transmitted towards the skin. This window defines the treatment area of the IPL hair removal device. By providing the light detecting arrangement outside the treatment area, anatomical structures within the treatment area may be imaged at greater depths.

Preferably, the light source 140 is configured to emit high-intensity light, so that, during use, the emitted light penetrates deep into body tissue. The light emitted by the light source may include light of at least one near-infrared wavelength (e.g. light having at least one wavelength in the range 800-1000 nm). For instance, the light source of an IPL hair removal device typically emits light with a 560-1200 nm spectral content.

The at least one light detector 151, 152 of the light detector arrangement 150 may comprise any detector capable of detecting near-infrared light. For instance, the at least one light detector may comprise at least one CMOS sensor, at least one CCD sensor and/or at least one SPAD sensor.

During normal operation of the handheld personal care device 120 by a subject (e.g. during a hair removal procedure in the case of an IPL hair removal device), the light source 140 is configured to emit light towards the subject's skin. The light emitted by an IPL hair removal device is pulsed light (typically pulses having a duration of a few milliseconds), but the invention is not limited to pulsed light. For instance, the light emitted by the light source may be continuous wave light (e.g. modulated light). Light that penetrates into the subject's skin and is backscattered by an anatomical structure 110 is detected by the light detecting arrangement 150.

In Fig. 1, the anatomical structure 110 is a blood vessel in a leg of a subject. The imaging of blood vessels in the leg is particularly valuable in the early detection of conditions such as deep vein thrombosis, varicose veins and peripheral artery disease. However, the invention may be used to image other anatomical structures, in particular, blood vessels in other areas of the body, such as the hand, under the tongue, etc.

The processing system 130 is configured to obtain, from the light detecting arrangement 150, imaging data 155 relating to light detected by the light detecting arrangement. The imaging data comprises one or more locations (relative to the light source 140) on the light detecting arrangement at which light emitted by the light source and backscattered by the anatomical structure 110 is detected by the light detecting arrangement. For instance, the imaging data may comprise which detector(s) and/or which pixel(s) of the at least one detector the backscattered light is detected at.

The processing system 130 is then configured to process the imaging data to determine, for at least one of the one or more locations, a relative depth at which light detected at the location was backscattered by the anatomical structure 110. The processing system may determine a relative depth for each location at which backscattered light was detected, or for a subset of depths (e.g. in the case of longitudinal monitoring, as described below, the processing system may determine a relative depth only for locations in which a change is detected).

In other words, the processing system 130 is configured to convert a distance between the light source 140 and the location at which backscattered light is detected to a relative depth at which the light was backscattered.

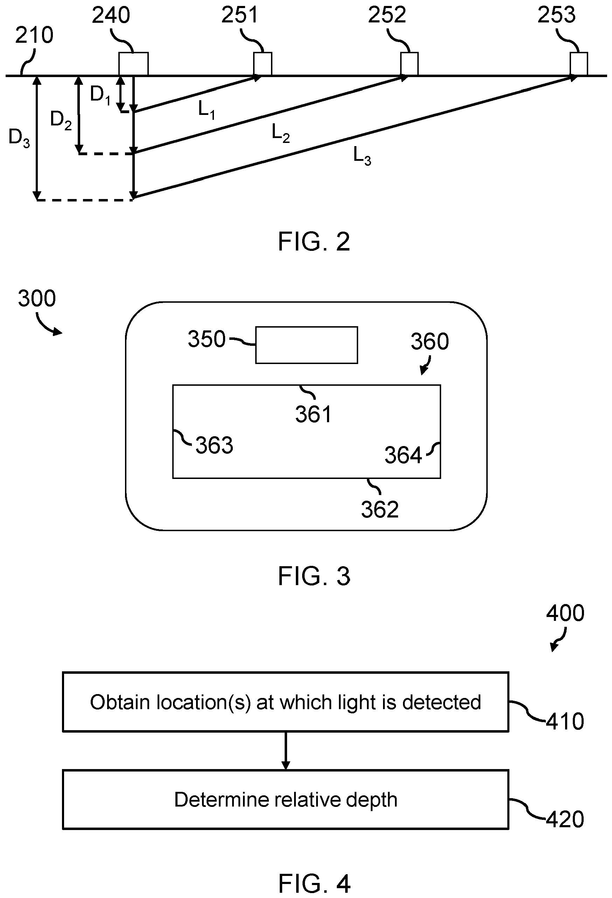

This concept is illustrated in Fig. 2, which shows the relationship between the distance between a light source and a light detector and the depth at which light detected at the light detector was backscattered by body tissue.

Light L₁ emitted by light source 240 towards the skin 210 of a subject and backscattered by body tissue at a shallow depth D₁ beneath the skin re-emerges from the skin close to the light source, and so is detected by a first detector 251 closest to the light source. Light L₂ backscattered at a greater depth D₂ re-emerges from the skin further from the light source, and so is detected by a second detector 252, which is positioned further from the light source than the first detector. Light L₃ backscattered at an even greater depth D₃ re-emerges from the skin further still from the light source, and so is detected at a third light detector 253, which is positioned further from the light source than the second detector.

As the skilled person will appreciate, the interpretation of backscattered light becomes more complicated the greater the size of the light source in the direction of the at least one light detector. Light detected at a single location on the light detecting arrangement may be the result of light emitted close to the detection location and backscattered from a shallow depth, or light emitted further from the detection location and backscattered from a greater depth.

In order to reduce the effect of the size of the light source, the light detecting arrangement may be positioned to reduce the variation in the distance between the at least one detector and a point at which light is emitted by the light source.

For instance, Fig. 3 illustrates a skin-contacting portion 300 of an IPL hair removal device, according to an embodiment of the invention. Light emitted by the light source of the IPL hair removal device is transmitted through a window 360, which has two first, longer edges 361, 362 and two second, shorter edges 363, 364. The light detecting arrangement 150 is provided at a side of longer edge 361 of the window. In this way, the distance between the side of the light source closest to the light detecting arrangement and the side of the light source furthest from the light detecting arrangement is smaller than if the light detecting arrangement were provided at a side of one of the shorter edges.

Returning to Fig. 1, in some examples, the processing system 130 may simply determine an order of depth for backscattered light detected at different locations, based on an order of distance of the locations from the light source 140. This information may be used to distinguish between anatomical structures from which the light was backscattered (e.g. distinguishing between a surface vein and a deep vein).

In other examples, the processing system 130 may determine a value of the depth relative to the light source 140. In order to determine a value of the depth, the processing system may use reference data relating to the relationship between each light detecting location of the light detecting arrangement (e.g. the location of each light detector and/or the location of each pixel of each light detector) and a depth of backscattered light detected at the location. The reference data may, for example, comprise data derived from Monte Carlo simulations of diffuse reflectance spectroscopy for the anatomical structure being imaged (see, for example, Kurakina et al. (2022), "Probing depth in diffuse reflectance spectroscopy of biotissues: a Monte Carlo study", Laser Phys. Lett., 19:035602).

The depth of light at which light detected at a particular light detecting location was backscattered is wavelength-dependent. In some examples, therefore, the imaging data 155 may further comprise a wavelength of light detected at each location for which backscattered light is detected. For instance, the imaging data may comprise a spectrum of detected light for each location, and the processing system 130 may process the spectrum and the location to determine the depth. In some examples, the light detecting arrangement may comprise light detectors provided at a same distance with respect to the light source on opposing sides of the light source, and the spectrum for each location relative to the light source may comprise an averaged spectrum from each light detector at a same distance from the light source.

The wavelength (or spectrum) of detected light may, for example, be determined by providing a wavelength filter between the anatomical structure 110 and each light detector 151, 152, so that different pixels of the light detector(s) detect different wavelengths of light. For example, the wavelength filter may be a concentric wavelength filter, which not only allows the wavelength(s) of detected light to be determined, but also enables the provision of more precise information about the relative depth of the backscattering of the light, by measuring differentially on more than one location of each light detector.

In some examples, a CMOS sensor or CCD sensor may be used in combination with a diffraction grating, such that each column of the sensor array detects a different wavelength range, in order to acquire spectrographic measurements. The overall wavelength range detected by the sensor would then depend on the diffraction grating used, while the resolution would be determined by the pixel pitch, slit width and grating density. The CMOS or CCD sensor and diffraction grating used with the sensor may be selected such that only first-order diffraction peaks within a desired wavelength range are captured.

The readings for each pixel in a column may be integrated and averaged to provide a light intensity measurement for the wavelength range detected by the column. This averaging accounts for any variation in the spatial uniformity of the light source 140.

The wavelength(s) of light detected at a location may not only improve a precision of a determination of the depth at which the light was backscattered, but may also enable classification of the anatomical structure 110 from which the light was backscattered. For instance, the processing system 130 may be configured to process the determined depth for a location and the wavelength(s) of light detected at the location to classify the anatomical structure at the determined depth. For example, in response to detecting red light at a detector location corresponding to a depth deep within the subject's body, the processing system may classify the anatomical structure as a deep artery, while blue light backscattered from a great depth may be classified as a deep vein.

In some examples, the time at which light is detected at a location may be used to more precisely determine the depth at which light was backscattered by the anatomical structure. The imaging data 155 may further comprise, for each location at which light is detected by the light detecting arrangement, a time at which light is detected at the location. The processing system 130 may determine the relative depth of backscattered light for a location at which the light was detected by processing the location and the time at which the light was detected at the location.

The use of the time at which the light was detected allows light emitted from a part of the light source further from the detection location (and therefore backscattered from a greater depth) to be distinguished from light emitted nearer to the detection location.

Where the at least one light detector 151, 152 of the light detecting arrangement 150 comprises at least one SPAD sensor, the time at which light is detected at a location may be measured at a sufficiently high resolution to reliably detect small changes in the depth at which light is backscattered, allowing variations in the size of the anatomical structure over time to be identified.

The processing system 130 may be configured to determine a change in the anatomical structure 110 by obtaining, for each depth, a corresponding historical depth at which light was backscattered by the anatomical structure, and processing each depth and corresponding historical depth.

A corresponding historical depth is a depth of the anatomical structure determined using light emitted from a same position relative to the anatomical structure (i.e. with the light source 140 at a same position relative to the anatomical structure). The corresponding historical depth may have been obtained during a previous operation of the handheld personal care device by the subject, and stored in a memory unit (not shown in Fig. 1).

Any suitable location/orientation sensing method may be used to determine the position of the light source 140 relative to the anatomical structure, in order to obtain the historical depth corresponding to the same light source position. For instance, the handheld personal care device 120 may further comprise a camera and an IMU sensor in order to track the position of the handheld personal care device relative to the subject.

In some examples, the imaging data 155 may comprise a histogram of photon arrival time for each pixel of at least one SPAD sensor. A shift in the histogram for a pixel, compared with a historical histogram for the same pixel obtained with a same light source position, indicates a change in the anatomical structure. The extent of the shift may be processed to determine a size of the change in the anatomical structure.

For instance, where the anatomical structure is a blood vessel, the processing system 130 may process a shift between a current histogram and a corresponding historical histogram to determine a change in blood vessel dilation.

In some examples, the processing system 130 may be further configured to predict whether a detected change in the anatomical structure is a dynamic change or a permanent change. For instance, several sets of measurements of backscattered light may be obtained at a same position of the light source 140 relative to the anatomical structure in a single measurement session (i.e. a single session of use of the handheld personal care device). A depth at which light was backscattered may be determined for at least one location at which backscattered light was detected for each set of measurements. In particular, a depth may be determined for each set of measurements for at least a location for which a change with respect to a historical depth has been identified.

The processing system 130 may compare the determined change with respect to the historical depth with variations in depth during the same measurement session in order to determine whether the determined change is a dynamic change (e.g. due to pulsatile blood flow) or a permanent change, which may be caused by disease. For instance, in response to a determination that the determined change is greater than any change in depth during the same measurement session, the processing system may determine that the change in the anatomical structure is permanent.

In some examples, the processing system 130 may alternatively use a predetermined threshold to determine whether a change in the anatomical structure is permanent or dynamic. For instance, in response to a difference between a current depth and a corresponding historical depth exceeding the predetermined threshold, the processing system may determine that the change in the anatomical structure is permanent. The predetermined threshold may correspond to an expected level of variation in the anatomical structure due to dynamic changes.

In response to a determination that a change in the anatomical structure is permanent, the processing system 130 may be configured to control an output user interface (not shown in Fig. 1) to provide a user-perceptible output of the determined change. In this way, the subject or the subject's clinician may be alerted at an early stage to changes in an anatomical structure that may be caused by disease.

In some examples, the processing system 130 may use the relative depths at which light was backscattered by the anatomical structure 110 to generate an image of the anatomical structure. The generated image may be stored in a memory unit and/or provided to an output user interface.

In order to obtain a fuller image of the anatomical structure, the generated image may be based on the relative depths of the anatomical structure determined at a plurality of different light source positions. For instance, the processing system 130 may repeat the steps of obtaining one or more locations on the light detecting arrangement 140 at which backscattered light is detected and determining one or more depths at which the light was backscattered as the handheld personal care device is moved along the subject's body during operation of the personal care device (e.g. as the IPL hair removal device of Fig. 1 is moved up the subject's leg). The processing system may then process the determined depths at each light source position to generate an image of the anatomical structure.

In some examples, the handheld personal care device 120 may further comprise a polarization conversion system and a polarizing filter (not shown in Fig. 1), in order to reduce an amount of surface reflection detected by the light detecting arrangement. The polarization conversion system is configured to intercept light emitted by the light source and convert p-polarized light to s-polarized light. For instance, the polarization conversion system may comprise a first polarizer provided over the light source 140 (e.g. in the IPL window 160) and a second polarizer perpendicular to the first polarizer and provided between the light source and the at least one light detector. The polarizing filter is positioned between the anatomical structure and the light detecting arrangement (during use), and is configured to filter out s-polarized light.

With this arrangement, surface-reflected light (i.e. light that is reflected from the surface of the subject's skin rather than penetrating into the skin) is s-polarized, and is therefore filtered out by the polarizing filter. Only p-polarized light that has been backscattered within the subject's body tissue will be detected by the light detecting arrangement.

Fig. 4 illustrates a computer-implemented method 400 for imaging an anatomical structure, according to an embodiment of the invention.

The method 400 begins at step 410, at which one or more locations, on a light detecting arrangement of a handheld personal care device, at which light emitted by a light source of the handheld personal care device and backscattered by the anatomical structure is detected by the light detecting arrangement, are obtained. The light detecting arrangement is provided at a known position relative to the light source.

At step 420, at least the location at which light is detected is processed, for at least one of the one or more locations, to determine a relative depth at which the light was backscattered by the anatomical structure.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs). Thus, the processing system may be embodied as a digital and/or analog processing system.

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system (100) for imaging an anatomical structure (110), the system comprising:
a handheld personal care device (120), comprising:
a light source (140) configured to emit light; and
a light detecting arrangement (150) comprising at least one light detector (151, 152) at a known position relative to the light source; and
a processing system (130) configured to:
obtain, from the light detecting arrangement, one or more locations on the light detecting arrangement, at which light emitted by the light source and backscattered by the anatomical structure is detected by the light detecting arrangement; and
for at least one of the one or more locations at which light is detected by the light detecting arrangement, process at least the location to determine a relative depth at which the light was backscattered by the anatomical structure.

2. The system (100) of claim 1, wherein:
the processing system (130) is further configured to obtain, for each location at which light is detected by the light detecting arrangement (150), a time at which light is detected at the location; and
the step of determining the depth at which the light was backscattered by the anatomical structure (110) further comprises processing at least, for each location for which a relative depth is determined, the time at which light is detected at the location.

3. The system (100) of claim 1 or 2, wherein the at least one light detector (151, 152) comprises at least one SPAD sensor.

4. The system (100) of any of claims 1 to 3, wherein:
the processing system (130) is further configured to obtain, for each location at which light is detected by the light detecting arrangement, a spectrum of light detected at the location; and
the step of determining the relative depth at which the light was backscattered by the anatomical structure (110) further comprises processing, for each location for which a relative depth is determined, the spectrum of light detected at the location.

5. The system (100) of claim 4, wherein the processing system (130) is further configured to process, for each location for which a relative depth is determined, the relative depth at which the light was backscattered by the anatomical structure (110) and the spectrum of light to classify the anatomical structure from which the light was backscattered.

6. The system (100) of any of claims 1 to 5, wherein the system further comprises:
a polarization conversion system configured to intercept light emitted by the light source and convert p-polarized light to s-polarized light; and
a polarizing filter provided over the light detecting arrangement to filter out s-polarized light.

7. The system (100) of any of claims 1 to 6, wherein the processing system (130) is further configured to:
repeat, for a plurality of positions of the light source (140) relative to the anatomical structure (110), the steps of:
obtaining, from the light detecting arrangement (150), one or more locations on the light detecting arrangement, at which light emitted by the light source and backscattered by the anatomical structure is detected by the light detecting arrangement; and
for at least one of the one or more locations at which light is detected by the light detecting arrangement, processing at least the location to determine a relative depth at which the light was backscattered by the anatomical structure; and
process each relative depth to generate an image of the anatomical structure.

8. The system (100) of any of claims 1 to 7, wherein the processing system (130) is further configured to:
obtain, for each relative depth, a corresponding historical depth at which light was backscattered by the anatomical structure (110), wherein the corresponding historical depth was determined using light emitted from a same position relative to the anatomical structure; and
process each relative depth and corresponding historical depth to determine, if present, a change in the anatomical structure.

9. The system (100) of claim 8, wherein the processing system (130) is further configured to:
repeat, at a same position of the light source (140) relative to the anatomical structure (110), the steps of:
obtaining, from the light detecting arrangement (150), one or more locations on the light detecting arrangement at which light emitted by the light source and backscattered by the anatomical structure is detected by the light detecting arrangement; and
for at least one of the one or more locations at which light is detected by the light detecting arrangement, processing at least the location to determine a relative depth at which the light was backscattered by the anatomical structure; and
process the determined relative depths and the determined change in the anatomical structure to predict whether, if present, the change is a dynamic or permanent change.

10. The system (100) of any of claims 1 to 9, wherein:
the handheld personal care device (120) is an intense pulsed light, IPL, hair removal device; and
the light source (140) is the intense pulsed light source of the IPL hair removal device.

11. The system (100) of claim 10, wherein:
the IPL hair removal device (120) comprises a window (160, 360) through which light emitted by the light source (140) is transmitted, the window comprising two first edges (361, 362) of a first length and two-second edges (363, 364) of a second, shorter length; and
the light detecting arrangement (150, 350) is provided at a side of one or both of the first edges.

12. The system (100) of any of claims 1 to 11, wherein the light detecting arrangement (150) is provided outside a tissue treatment area of the handheld personal care device (120).

13. The system (100) of any of claims 1 to 12, wherein the anatomical structure (110) is a blood vessel.

14. A computer-implemented method (400) for imaging an anatomical structure (110), the computer-implemented method comprising:
obtaining, from a light detecting arrangement (150) of a handheld personal care device (120), one or more locations, on the light detecting arrangement, at which light emitted by a light source (140) of the handheld personal care device and backscattered by the anatomical structure is detected by the light detecting arrangement, wherein the light detecting arrangement is provided at a known position relative to the light source; and
for at least one of the one or more locations at which light is detected by the light detecting arrangement, process at least the location to determine a relative depth at which the light was backscattered by the anatomical structure.

15. A computer program product comprising computer program code means which, when executed on a computing device having a processing system, cause the processing system to perform all of the steps of the computer-implemented method (400) according to claim 14.
